(19) Europäisches Patentamt
European Patent Office
Office européen des brevets

(11) EP 0 505 640 B2

(12) NEW EUROPEAN PATENT SPECIFICATION

(45) Date of publication and mention of the opposition decision:
**27.02.2002 Bulletin 2002/09**

(51) Int Cl.[7]: **A61K 38/55**, A61K 31/505, A61P 35/00

(45) Mention of the grant of the patent:
**23.08.1995 Bulletin 1995/34**

(21) Application number: **91308457.0**

(22) Date of filing: **17.09.1991**

# (54) Improved therapeutic method

Verbesserte Therapiemethode

Méthode thérapeutique améliorée

(84) Designated Contracting States:
**AT BE CH DE DK ES FR GB GR IT LI LU NL SE**

(30) Priority: **29.03.1991 US 677031**

(43) Date of publication of application:
**30.09.1992 Bulletin 1992/40**

(73) Proprietor: **ELI LILLY AND COMPANY**
**Indianapolis, Indiana 46285 (US)**

(72) Inventors:
- **Grindey, Gerald Burr**
  **Indianapolis, Indiana 46250 (US)**
- **Chuan, Shih**
  **Carmel, Indiana 46032 (US)**

(74) Representative: **Burnside, Ivan John et al**
**Eli Lilly and Company Limited Lilly Research Centre Erl Wood Manor**
**Windlesham, Surrey GU20 6PH (GB)**

(56) References cited:

| | |
|---|---|
| **EP-A- 0 367 902** | **EP-A- 0 407 212** |
| **US-A- 4 931 441** | **US-A- 4 999 424** |

- **Cancer Progress Conference, New York, 18 March, 1991**
- **Cancer Research, 33, 1729-1734 (July 1973) entitled Improved Therapeutic Index of Methotrexate with "Leucovorin Rescue"**
- **The Merck Index, 10th edition, 1983, p. 4114, Entry No. 4111 entitled Folinic Acid**
- **Goodman and Gilman's "The Pharmacological Basis of Therapeutics", seventh edition, 1985, pp. 1263-1267**
- **Abstract and Poster No. 1800 entitled 'Murine Liver Folate Binding Protein is a High Affinity Novel Isoform of the Folate Receptor' by KA Shackelford et al., Lilly Research Laboratories; Meeting of April, 1994, of The American Association for Cancer Research**
- **Arthritis and Rheumatism, vol 33, No. 1, January 1990**
- **Proceedings of the Annual Meeting of the American Society of Clinical Oncology, 1990, vol 9; Abstract 293**
- **European J. Cancer, 17, 11-19(1981), entitled A Potent Antitumour Quinazoline Inhibitor of Thymidylate Synthase: Synthesis, Biological Properties and Therapeutic Results in Mice by T R Jones et al.**
- **British Journal of Cancer, Supplement XIII, Vol 63, May, 1991, relating to a Meeting held 14-17th April, 1991, Abstract No. 66**
- **J. Clin. Oncol., 1989, 7, 1419-1426 entitled The modulation of fluorouracil with leucovorin in metastatic colorectal carcinoma: a prospective randomized Phase III trial**
- **Goodman and Gilman's "The Parmacological Basis of Therapeutics", seventh edition, 1985, pp. 1332-1333**
- **Laboratory Investigation, 1989, vol 60, pp. 737-746**
- **Biochemical Pharmacology, 1979, 28, pp. 2993-2997**
- **J. Biol. Chem., 1978, 253, p. 242**
- **Proceedings of The American Association for Cancer Research, volume 32, March 1991, Abstract No. 1921**
- **A. I. Pavlotsky et al., Cancer Treatment Rep., vol 61, no. 5, (1977) pp. 895-897**



Printed by Jouve, 75001 PARIS (FR)

(Cont. next page)

- S. L. Morgan et al., Arthritis and Rheumatism, vol 33, no. 1 (1990), pp. 9-18
- G. B. Grindey et al., Proc. Am. Assoc. Cancer Res., Abs. 1921, vol 32 (1991), p. 324
- M. A. Kane et al., Laboratory Investigation, vol 60, no. 6 (1989), pp. 737-746
- H. M. Said et al., Cancer Chemother. Pharmacol., vol 16, (1986), pp. 121-124
- R. C. Jackson et al., Chapter 8 of "Folate Antagonists as Therapeutic Agents", Academic Press Inc. (1984), pp. 289-315
- Physicians' Desk Reference, 45th Edition, Medical Economics Company Inc. (1991), pp. 776, 1185-1189
- ABPI Data Sheet Compendium, Datapharm Publications Limited (1990), pp. 824/825, 838-840
- G. P. Beardsley et al., Proceedings of the 9th International Symposium on Pteridines and Folic Acid Derivatives, Zurich, 3-8 September 1989, pp. 1001-1008
- G. Pizzorno et al., Proceedings of the 9th International Symposium on Pteridines and Folic Acid Derivatives, Zurich, 3-8 September 1989, pp. 1031-1034

## Description

This invention relates to the use of folic acid for reducing the toxicity, but not the therapeutic effect, of antitumor antifolate agents.

Lometrexol is the generic name given to 5,10-dideazatetrahydrofolic acid, also referred to as DDATHF. Lometrexol is a member of a new class of antitumor agents which have been found to specifically inhibit glycinamide ribonucleotide (GAR) transformylase, an enzyme required in the initial stages of purine biosynthesis, see J. Med. Chem., 28, 914 (1985). Several of these GAR-transformylase inhibitors are described, along with their antitumor utilities, by Taylor et al. in U. S. Patent Nos. 4,684,653, 4,833,145, 4,902,796, 4,871,743 and 4,882,334. GAR-transformylase inhibitors are also known to be useful in treating conditions such as gout, psoriasis, mycosis fungoides, autoimmune disorders, rheumatoid arthritis and other inflammatory disorders, and during organ transplantation and other related immunosuppressant related conditions.

Lometrexol has been studied clinically and shown to be a potent antitumor agent, especially against solid tumors such as colorectal, lung, breast, head and neck and pancreatic; Young et al., Proc. Amer. Assoc. Cancer Research, 31, 1053 (1990). Like most other antitumor agents, Lometrexol exhibits some undesirable side effects, in addition to its efficacy against tumors; Muggia et al., Proc. Amer. Soc. Clinical Oncology, 9, 1285 (1990). Typical side effects observed to date include anorexia, weight loss, mucositis, leukopenia, anemia, hypoactivity and dehydration.

We have now discovered that the toxic effects of lometrexol and related GAR-transformylase inhibitors and thymidylate synthase (TS) inhibitors which bind to folate binding protein (FBP) (see, e.g., kane, et al., Laboratory Investigation, 60, 737 (1989)) can be significantly reduced by the presence of folic acid or a physiologically-available salt or ester thereof, without adversely affecting therapeutic efficacy. The present invention thus provides a method for improving the therapeutic utility of GAR-transformylase inhibitors and thymidylate synthase (TS) inhibitors by coadministering folic acid or a physiologically-available salt or ester thereof to the host undergoing treatment.

In one aspect of this invention, we provide a method of inhibiting the growth of GAR-transformylase-dependent tumors in mammals comprising administering to said mammals an effective amount of a GAR-transformylase or TS of folic acid inhibitor which binds to a FBP in combination with a toxicity-reducing amount of folic acid or a physiologically-available salt or ester thereof. The invention more particularly provides a method for reducing the mammalian toxicity of a GAR-transformylase or TS inhibitor which binds to a FBP which comprises administering a toxicity-reducing amount of folic acid or a physiologically-available salt or ester thereof to the mammal receiving treatment. In particular, there is provided a method for reducing the toxicity of a GAR-transformylase or TS inhibitor which binds to a FBP in a mammal which comprises pretreating the mammal with an amount of folic acid, or a physiologically-available salt or ester thereof, sufficient to have substantially blocked the FBP before administration of the antifolate. In the most preferred embodiment of the invention, Lometrexol is administered to a subject suffering from a solid tumor or other type of cancer and in need of treatment after pretreatment with folic acid, thereby reducing toxic effects of Lometrexol while maintaining good antitumor activity.

The invention provides a method for reducing the toxicity of GAR-transformylase inhibitors or thymidylate synthase (TS) inhibitors that bind to a FBP that is found in biological systems by the prior administration of folic acid or a physiologically-available salt or ester thereof. GAR-transformylase inhibitors are those compounds which effectively inhibit the biological actions of the enzyme known as glycinamide ribonucleotide transformylase. This enzyme is well known to be required in the initial stages of purine biosynthesis in mammals, which is implicated in DNA synthesis. Interruption of this biosynthetic pathway causes a disturbance in DNA synthesis and consequently causes cell death. Any compound which is shown to inhibit the GAR-transformylase is subject to treatment in accordance with this invention.

Typical GAR-transformylase inhibitors include the pyrido[2,3-d]pyrimidine derivatives described by Taylor et al. in U.S. Patent Nos. 4,684,653, 4,833,145, 4,902,796, 4,871,743 and 4,882,334. Another series of GAR-transformylase inhibitors has recently been described by Akimoto in U.S. Patent No. 4,997,838. Antifolate compounds which can be employed in this invention include thymidylate synthase inhibitors as found in EPO Patent Application 239,362. Other GAR-transformylase inhibitors are also included within the scope of this invention, and such compounds can be determined by routine evaluation of either their ability to interact with and inhibit the subject enzyme or to bind to the FBP.

In a preferred embodiment of the invention, folic acid is administered to a subject subsequently receiving an agent defined by the formula

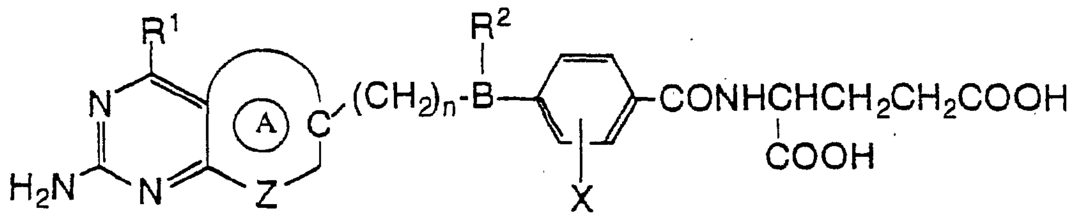

wherein

$R^1$ is hydroxy or amino;
$R^2$ is hydrogen, methyl, ethyl,or propynyl;
B is -CH- or -N-;
n is 1, 2 or 3;
Z is nitrogen or carbon;
A is pyrido, tetrahydropyrido, pyrrolo, dihydropyrrolo, cyclopentyl or cyclohexyl;
X is hydrogen or halo;

and pharmaceutically acceptable salts thereof.

In a particularly preferred embodiment of the invention, lometrexol Is utilized as the GAR-transformylase inhibitor.

As noted above, the drug products which can be employed in the present invention include other GAR-transformylase or TS inhibitors which are capable of binding to folate binding protein. Folic acid itself has a binding constant (ng/ml) of 1.8, and lometrexol has a binding constant of 9.7 to bovine FBP. Any GAR-transformylase or TS inhibitor that binds at less than about 500 ng/ml can be utilized in the method of this invention. The folate binding constant for drug products can be readily determined by the general procedure of Dunn and Foster, Clin. Chem., 19 (10), 1101-1105 (1973). Typical antifolates evaluated in the referenced procedure have the following folate binding constants presented in Table I below:

TABLE I

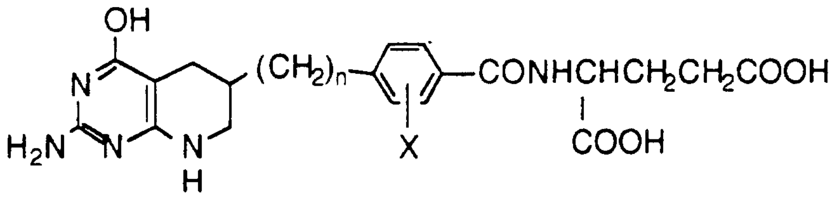

| | | Binding Constant (ng/ml) |
|---|---|---|
| n = 3 | X = H | 12.5 |
| n = 2 | X = 2-F | 14.0 |
| n = 2 | X = 3-F | 30.7 |
| 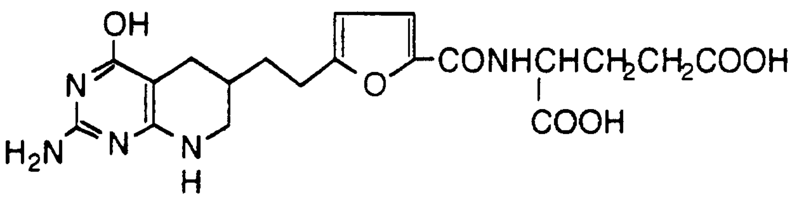 | | 250.8 |
| 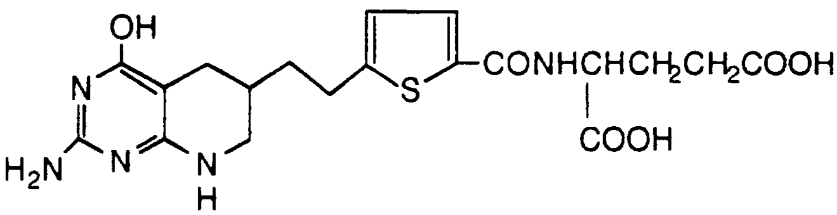 | | 24.5 |
| 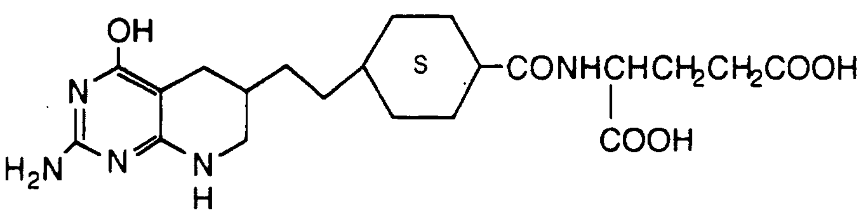 | | 26.0 |

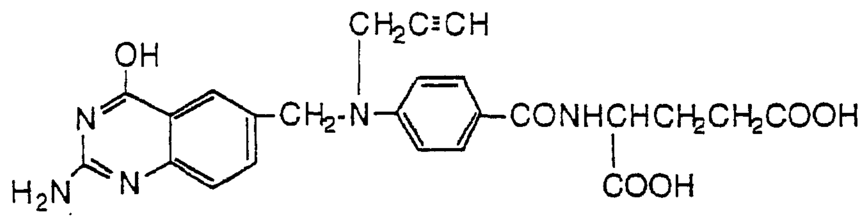

12.0

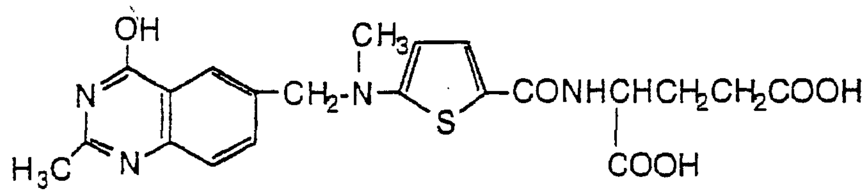

245

As used in this invention, the term "FBP binding agent" refers to foilc acid.

Folic acid is a vitamin which is required by mammals for proper regeneration of the blood-forming elements and their functioning, and as a coenzyme is involved in intermediary metabolic processes in which one-carbon units are transferred. These reactions are important in interconversions of various amino acids and in purine and pyrimidine synthesis. Folic acid is commonly supplied to diets of humans via consumption of food sources such as liver, kidney, dry beans, asparagus, mushrooms, broccoli, lettuce, milk and spinach, as well as by vitamin supplements. The minimum amount of folic acid commonly required by normal adults is about 0.05 mg/day. According to this invention, folic acid, or a physiologically-available salt or ester thereof, is administered to a human subject at a dose of 0.5 mg/day to 30 mg/day to diminish the toxic effects of a GAR-transformylase or TS inhibitor also being administered to such subject. In a preferred embodiment, folic acid will be administered at about 1 to 5 mg/day together with the normal dosing of GAR-transformylase inhibitor such as lometrexol.

"Physiologically-available salt" refers to potassium, sodium, lithium, magnesium, or preferably a calcium salt of the FBP binding agent "Physiologically-available ... ester" refers to esters which are easily hydrolyzed upon administration to a mammal to provide the corresponding FBP binding agent free acid, such as $C_1$-$C_4$ alkyl esters, mixed anhydrides, and the like.

The FBP binding agent to be utilized according to this Invention can be in its free acid form, or can be in the form of a physiologically-acceptable salt or ester which is converted to the parent acid in a biological system. The dosage generally will be provided in the form of a vitamin supplement, namely as a tablet administered orally, preferably as a sustained release formulation, as an aqueous solution added to drinking water, an aqueous parenteral formulation, e.g., an intravenous formulation, or the like.

The FBP binding agent is administered to the subject prior to treatment with the GAR-transformylase or TS inhibitor. Pretreatment with the suitable amount of FBP binding agent from about 1 to about 24 hours is usually sufficient to substantially bind to and block the folate binding protein prior to administration of the GAR-transformylase or TS inhibitor. Although one single dose of the FBP binding agent, preferably an oral administration of folic acid, should be sufficient to load the folate binding protein, multiple dosing of the FBP binding agent can be employed for periods up to weeks before treatment with the active agent to ensure that the folate binding protein is sufficiently bound in order to maximize the benefit derived from such pretreatment.

In the especially preferred embodiment of this invention, 1 mg to 5 mg of folic acid is administered orally to a human subject 1 to 24 hours prior to the parenteral administration of the amount of lometrexol which is normally required to attain the desired therapeutic benefit. Although greater or additional doses of folic acid are also operable, the above parameters will usually bind the folate binding protein in an amount sufficient to reduce the toxicity effects normally seen upon lometrexol administration above.

It should be noted that the FBP binding agent is not an antitumor agent and that the pretreatment of a human subject with a FBP binding agent is not a synergistic or potentiating effect. Rather, by having substantially bound the folate binding protein with a FBP binding agent prior to administration of the GAR-transformylase or TS inhibitor, the toxic effects of such subsequent treatment are greatly reduced without affecting the therapeutic efficacy.

The effect of folic acid on GAR-transformylase inhibitors has been demonstrated in standard tests commonly utilized to determine the antitumor activity and toxic effects of the GAR-transformylase inhibitors themselves. In one such test, mice are inoculated with the C3H strain of mammary adenocarcinoma by inserting a 2 mm by 2 mm section

of tumor into the axillary region of the mice by trocar. In all experiments, lometrexol was administered intraperitoneally once a day for five consecutive days, starting on the day following tumor implantation. Ten animals were used at each dosage level. Antitumor activity was assessed on day ten by measuring the length and width of the tumor growth using vernier calipers, and the activity was expressed as a percent inhibition of tumor growth.

When lometrexol was administered to infected mice which are maintained on a diet totally free of folic acid for two weeks prior to and during treatment, it exhibited moderate antitumor activity at very low doses, but also caused severe toxicity at a very low dose (measured as death of mice). These data are presented in Table II below.

TABLE II

| Antitumor Activity and Toxicity of Lometrexol in C3H Mice after Two Weeks on Folate-Free Diet | | |
|---|---|---|
| Lometrexol Dose (mg/kg) | Antitumor Activity (% Inhibition) | Toxicity (Mice Dead/Total Mice) |
| 0.0625 | 0% | 0/10 |
| 0.125 | 0% | 0/10 |
| 0.25 | 21% | 0/10 |
| 0.5 | 88% | 0/10 |
| 1.0 | 100% | 8/10 |

A test group of mice were maintained on a folic acid free diet for two weeks before treatment. Folic acid was then administered during the treatment by providing the animals drinking water containing 0.0003% folic acid (weight/volume). This concentration translates to about 1.75 mg of folic acid per square meter of body surface per day, since the animals consume about 4 ml of water each day.

$$\frac{0.0003 \text{ grams}}{100 \text{ ml.}} \times \frac{4 \text{ ml.}}{\text{day}} = \frac{0.000012 \text{ grams}}{\text{day}} = \frac{0.012 \text{ milligrams}}{\text{day}}$$

The average size of a mouse is 0.00687 $m^2$

$$\frac{0.012 \text{ grams}}{\text{day}} \times \frac{1}{0.00687 \text{ m}^2} = 1.75 \text{ mg/m}^2\text{/day}$$

For a human subject of about 1.73 $m^2$ size, this translates to an adult human dosage of about 3.0 mg/day. The effect of the foregoing folate dosage on the activity and toxicity of lometrexol is shown in Table III below:

TABLE III

| Antitumor Activity and Toxicity of Lometrexol in C3H Mice after Two Weeks on Folate-Free Diet Plus Addition of 0.0003% Folate to Drinking Water | | |
|---|---|---|
| Lometrexol Dose (mg/kg) | Antitumor Activity (% Inhibition) | Toxicity (Mice Dead/Total Mice) |
| 0.125 | 13% | 0/10 |
| 0.25 | 26% | 0/10 |
| 0.5 | 48% | 0/10 |
| 1.0 | 97% | 0/10 |
| 2.0 | 98% | 0/10 |
| 4.0 | 99% | 4/10 |

As the foregoing results indicate, addition of the indicated level of folic acid to the diet of a subject receiving lometrexol results in excellent antitumor activity at low doses, with little or no toxic effects.

Larger doses of folic acid appear to have an even more dramatic effect on the antitumor activity and toxicity of the GAR-transformylase inhibitor. For example, when mice were maintained on a folate acid-free diet for two weeks before treatment with lometrexol, and then given water containing 0.003% (weight/volume) of folic acid (which translates to an adult human dose of about 30 mg/day), good antitumor activity of lometrexol is observed at higher dose levels. These results are shown in Table IV below:

TABLE IV

| Antitumor Activity and Toxicity of Lometrexol in C3H Mice after Two Weeks on Folate-Free Diet Plus Addition of 0.003% Folate to Drinking Water | | |
|---|---|---|
| Lometrexol Dose (mg/kg) | Antitumor Activity (% Inhibition) | Toxicity (Mice Dead/Total Mice) |
| 6.25 | 91 % | 0/10 |
| 12.5 | 89% | 0/10 |
| 25 | 97% | 0/10 |
| 50 | 96% | 0/10 |

The foregoing data establish that for tumor bearing mice maintained on a folic acid free diet prior to and during treatment with lometrexol, the toxicity of lometrexol is very large, with 1 mg/kg/day being lethal to the majority of the mice, and lower antitumor activity is observed at non-toxic drug doses. Very low doses of folic acid (about 1 to 2 mg/day for an adult human) partially reversed drug toxicity and improved antitumor activity. Larger doses of folic acid (up to about 30 mg/day for an adult human) dramatically reduced lometrexol toxicity and markedly improved antitumor activity. Thus, the use of folic acid in combination with a GAR-transformylase inhibitor markedly reduces drug toxicity without adversely affecting antitumor activity.

In a typical clinical evaluation involving cancer patients, all of whom have histologically or cytologically confirmed diagnosis of cancer, lometrexol is administered in combination with folic acid. Lometrexol is administered in four doses over a two week period by rapid intravenous injection, followed by two weeks of non-therapy. Dosing is made on days 1, 4, 8 and 11 of any two week period. Patients will have an initial course of therapy at a dose of 5 $mg/m^2$/dose, and depending upon the toxic effects observed in the initial course, their subsequent courses may be at the same dose, or may be escalated to 6 $mg/m^2$, or may be attenuated to 4 $mg/m^2$.

These patients will also receive orally 1 mg/day of folic acid, beginning the day before they are started on the first course of lometrexol, and continuing throughout their exposure to the drug. Such dosage of folic acid will be given once daily, generally in the morning hours.

In preparation for the foregoing clinical study, pilot studies in humans have established that folic acid given to patients receiving lometrexol has effected reduced side effects due to the lometrexol. Specifically, in one subject who had a nasalpharyngeal carcinoma, who was supplimented with folic acid at 0.5 to 1.0 mg/day, lometrexol was well tolerated for up to 12 months of therapy. Moreover, this patient has no clinical evidence of disease after the 12 months of therapy. These data are consistent with the animal studies reported above.

## Claims

1. The use of folic acid, or a physiologically-available salt or ester thereof, in the preparation of a medicament useful in lowering the mammalian toxicity of an antifolate which binds to a folate binding protein, said antifolate being administered for the treatment of a tumor, wherein the antifolate is a GAR-transformylase inhibitor or a thymidylate synthase inhibitor, and the medicament is for administration to a human subject prior to said antifolate, and in an amount to provide a dosage of up to 30mg folic acid per day.

2. The use according to claim 1, wherein the antifolate is a GAR-transformylase inhibitor of the formula

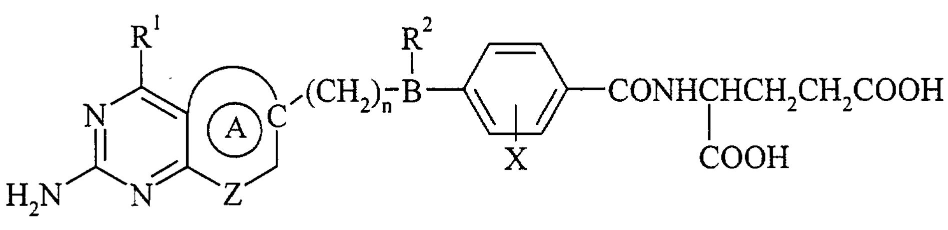

wherein

$R^1$ is hydroxy or amino;
$R^2$ is hydrogen, methyl, ethyl or propynyl;

B is -CH- or -N-;
nis 1, 2 or 3;
Z is nitrogen or carbon;
A is pyrido, tetrahydropyrido, pyrrolo, dihydropyrrolo, cyclopentyl or cyclohexyl;
X is hydrogen or halo;

or a pharmaceutically acceptable salt thereof.

3. The use according to claim 1, wherein the antifolate is

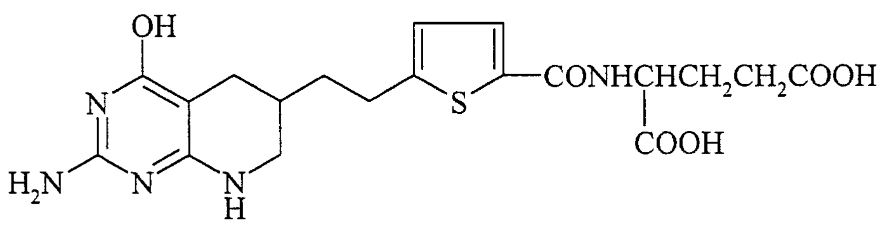

4. The use according to claim 1, wherein the antifolate is lomotrexol.

5. The use according to claim 1, wherein the dosage of folic acid is from 0.5 to 30 mg/day.

6. A combination of folic acid, or a physiologically-available salt or ester thereof, and lomotrexol, for use in cancer chemotherapy.

**Patentansprüche**

1. Verwendung von Folsäure oder einem physiologisch verfügbaren Salz oder Ester hiervon bei der Herstellung eines Arzneimittels, das geeignet ist zur Absenkung der Säugetiertoxizität eines Antifolats, das an ein folatbindendes Protein bindet, wobei dieses Antifolat zur Behandlung eines Tumors verabreicht wird, worin das Antifolat ein GAR-Transformylase-Inhibitor oder ein Thymidylatsynthase-Inhibitor ist und das Arzneimittel vor dem Antifolat und in einer Menge verabreicht wird, daß sich eine Dosis von bis zu etwa 30 mg Folsäure pro Tag ergibt.

2. Verwendung nach Anspruch 1, worin das Antifolat ein GAR-Transformylase-Inhibitor der folgenden Formel

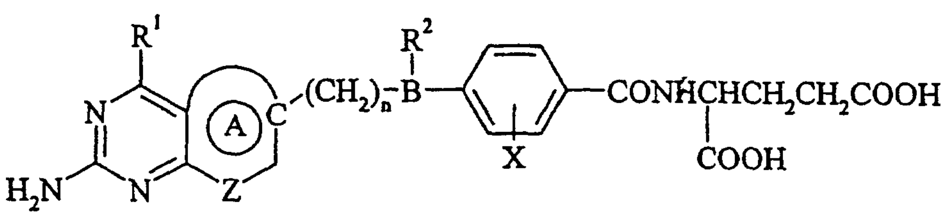

worin

$R^1$ ein Hydroxy- oder Aminorest ist,
$R^2$ Wasserstoff, ein Methyl-, Ethyl- oder Propinylrest ist,
B für -CH- oder -N- steht,
n für 1, 2 oder 3 steht,
Z Stickstoff oder Kohlenstoff ist,
A ein Pyrido-, Tetrahydropyrido-, Pyrrolo-, Dihydropyrrolo-, Cyclopentyl- oder Cyclohexylrest ist,
X Wasserstoff oder Halogen ist,

oder ein pharmazeutisch annehmbares Salz hiervon ist.

3. Verwendung nach Anspruch 1, worin das Antifolat die folgende Formel hat

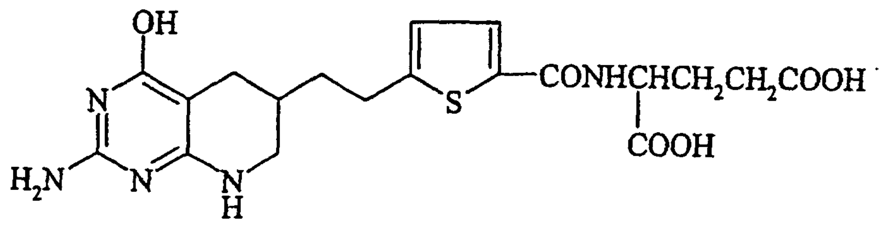

4. Verwendung nach Anspruch 1, worin das Antifolat Lomotrexol ist.

5. Verwendung nach Anspruch 1, worin die Dosis der Folsäure 0,5 bis 30 mg pro Tag beträgt.

6. Kombination aus Folsäure oder einem physiologisch verfügbaren Salz oder Ester hiervon und Lomotrexol zur Verwendung bei einer Krebs-Chemotherapie.

## Revendications

1. Utilisation d'acide folique ou d'un de ses sels ou de ses esters physiologiquement disponibles, dans la préparation d'un médicament utile dans la diminution de la toxicité mammalienne d'un antifolate qui se lie à une protéine de liaison au folate, ledit antifolate étant administré pour le traitement d'une tumeur, dans laquelle l'antifolate est un inhibiteur de la GAR-transformylaser ou un inhibiteur de la thymidylate synthase, et le médicament est destiné à une administration à un sujet humain avant ledit antifolate et en une quantité telle que l'on obtient une posologie allant jusqu'à 30 mg d'acide folique par jour.

2. Utilisation selon la revendication 1, dans laquelle l'antifolate est un inhibiteur de la GAR-transformylase répondant à la formule

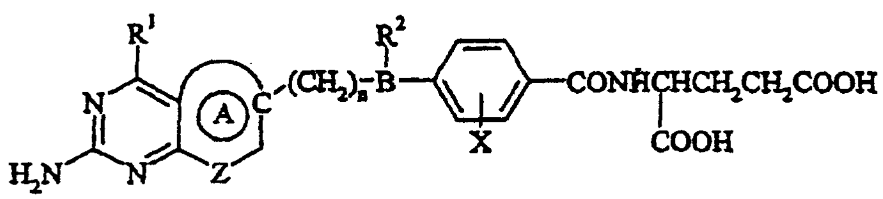

dans laquelle

$R^1$ représente un groupe hydroxyle ou un groupe amino ;
$R^2$ représente un atome d'hydrogène, un groupe méthyle, un groupe éthyle ou un groupe propynyle ;
B représente un groupe -CH- ou un groupe -N- ;
n représente 1, 2 ou 3 ;
Z représente un atome d'azote ou un atome de carbone ;
A représente un groupe pyrido, un groupe tétrahydropyrido, un groupe pyrrolo, un groupe dihydropyrrolo, un groupe cyclopentyle ou un groupe cyclohexyle ;
X représente un atome d'hydrogène ou un atome d'halogène ;

ou un de ses sels pharmaceutiquement acceptables.

3. Utilisation selon la revendication 1, dans laquelle l'antifolate est

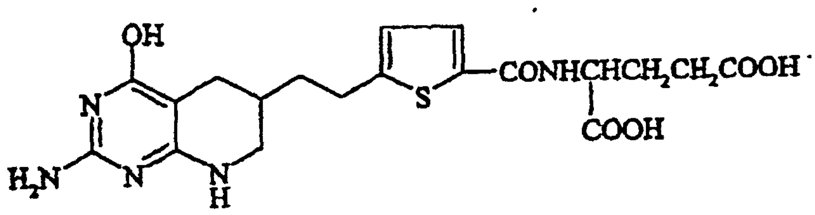

**4.** Utilisation selon la revendication 1, dans laquelle l'antifolate est le lomotrexol.

**5.** Utilisation selon la revendication 1, dans laquelle la posologie de l'acide folique s'élève de 0,5 à 30 mg/jour.

**6.** Combinaison d'acide folique, d'un de ses sels ou d'un de ses esters physiologiquement disponibles et de lomotrexol à utiliser dans la chimiothérapie du cancer.